(19) Europäisches Patentamt
European Patent Office
Office européen des brevets

(11) EP 3 335 699 A1

(12) EUROPEAN PATENT APPLICATION

(43) Date of publication:
**20.06.2018 Bulletin 2018/25**

(21) Application number: **16204499.4**

(22) Date of filing: **15.12.2016**

(51) Int Cl.:
***A61K 9/14*** *(2006.01)*
***A61K 47/02*** *(2006.01)*
***A61K 9/16*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(71) Applicant: **H e x a l Aktiengesellschaft
83607 Holzkirchen (DE)**

(72) Inventors:
- **WOLF, Claudia
83607 Holzkirchen (DE)**
- **KREKELER, Andreas
83607 Holzkirchen (DE)**
- **SEDLMAYR, Michael
83607 Holzkirchen (DE)**

(74) Representative: **Prüfer & Partner mbB
Patentanwälte · Rechtsanwälte
Sohnckestraße 12
81479 München (DE)**

# (54) SELEXIPAG FORMULATION IN LIQUISOLID SYSTEM

(57) The present invention relates to a process for the preparation of a liquisolid powder, wherein the process comprises the steps of providing a solution comprising 2-{4-[N-(5,6-diphenylpyrazin-2-yl)-N-(isopropylamino]butoxy}-N-(methanesulfonyl)acetamide (selexipag) and a solvent, wherein said solvent is a non-volatile solvent, a mixture of non-volatile solvents, or a mixture of non-volatile and volatile solvents, to form a solution comprising selexipag; and mixing the solution thus obtained with a solid porous carrier, thereby obtaining a liquisolid powder.

The present invention further refers to a process for the preparation of a solid final dosage form, comprising the additional step of formulating the liquisolid powder in a final dosage form.

The present invention also relates to a liquisolid powder comprising selexipag, a final dosage form comprising the liquisolid powder, and the use thereof in a method of prevention or treatment of pulmonal arterial hypertension.

Fig. 1

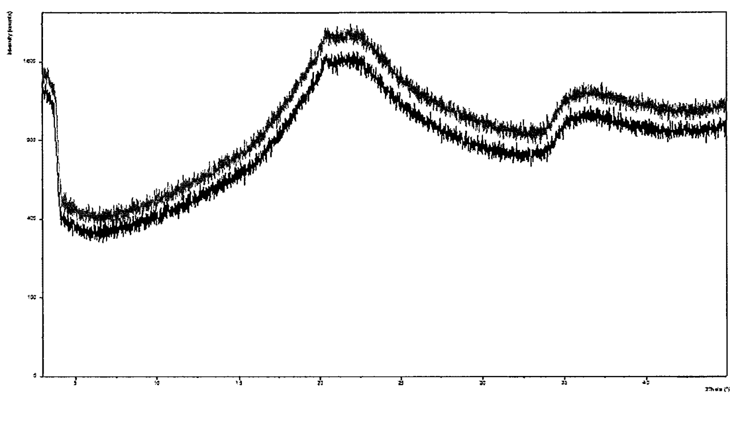

Legend:
upper curve (light grey): samples of Example 5
lower curve (dark grey): sample of Example 5 stored at 20-25°C; in closed HDPE bottles for 6 weeks

EP 3 335 699 A1

## Description

Field of the invention

The present invention belongs to the field of pharmaceutical industry and relates to a process for the preparation of a liquisolid powder, comprising 2-{4-[N-(5,6-diphenylpyrazin-2-yl)-N-(isopropylamino]butoxy}-N-(methanesulfonyl)acetamide (selexipag). The present invention further refers to a liquisolid powder comprising selexipag, to a final dosage form comprising said liquisolid powder, and to the preparation thereof. Finally, the present invention refers to said liquisolid powder for use in a method of prevention or treatment of pulmonal arterial hypertension.

Background of the invention

Prostaglandin I2 (PGI2) is produced from arachidonic acid in the living body and exhibits various pharmacological effects such as inhibition of platelet aggregation, vasodilation, inhibition of lipid deposition, and inhibition of leucocyte activity. Thus, it is assumed that PGI2 is effective for the treatment of various diseases that are in association with said states. However, PGI2 is not suitable for being used as a medicament, because it has severe disadvantages such as insufficient chemical stability and a biological half-life that is too short for being useful.

When compared to PGI2, 2-{4-[N-(5,6-diphenylpyrazin-2-yl)-N-(isopropylamino]butoxy}-N-(methanesulfonyl)acetamide (selexipag) (Compound I) exhibits improved properties and thus, generally, is useful as therapeutic agent for the above-cited diseases.

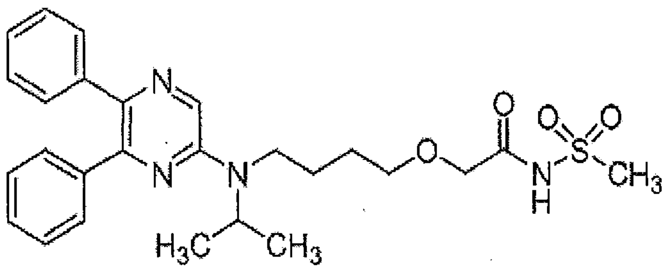

(I)

Selexipag has an excellent PGI2 agonistic effect and shows a platelet aggregation inhibitory effect, a vasodilative effect, a bronchiodilative effect, a lipid deposition inhibitory effect, a leukocyte activation inhibitory effect, and the like.

In prior art, various forms of selexipag, as well as formulations comprising selexipag, are known:

EP 2 447 254 discloses crystalline forms of selexipag, as well as the preparation thereof.

However, in general, crystalline forms of selexipag have the disadvantage that they are only poorly soluble, which is reflected by their classification as BCS (Biopharmaceutics Classification System) class IV, which means that they show low solubility and low permeability. Poorly soluble, or even non-soluble, active pharmaceutically ingredients (API) generally have the problem that they exhibit a poor, non-sufficient bioavailability upon administration to a subject in need thereof. This poor bioavailability is - inter alia - due to the poor dissolution profile of selexipag.

Since new formulation techniques are continuously being developed, more and more tools for formulating selexipag become available and a skilled person was motivated to develop new pharmaceutical dosage forms containing selexipag exhibiting improved properties, e.g. with regard to its solubility and dissolution profile, or with regard to its stability.

For instance, in order to improve the solubility of selexipag, dosage forms comprising amorphous selexipag in combination with a certain excipient (Soluplus®; a polyvinyl-caprolactam-polyvinyl-acetate-polyethylene glycol graft copolymer) have been developed (Research Disclosure, "Amorphous Selexipag Formulations", Research Disclosure database number 618027, 2015). However, amorphous forms of APIs generally can have problems with regard to their stability, e.g. because the amorphous forms tend to crystallize.

In order to develop stabilized amorphous selexipag, a person skilled in the art would predominantly consider formulation techniques where amorphous selexipag is directly formed in the presence of the stabilizing excipient, e.g., forming amorphous selexipag by spray-drying in the presence of Soluplus®. (see Research Disclosure, "Amorphous Selexipag Formulations", cited above). In view of the very poor solubility of selexipag, apparently, it is desirable to use formulation techniques where low concentration selexipag solution can be used, e.g., a spray-drying process where the high amount of solvent can be removed in the drying step and is not part of the final dosage form. Another technique

which could be contemplated is the use of a wet granulation process wherein the amorphous selexipag is present in solid form.

Despite the above described formulations, there is still a need and accordingly a problem, to provide formulations comprising selexipag with improved performance, e.g. with regard to solubility, dissolution, bioavailability, and use attributes.

Summary of the invention

The present invention provides the following aspects, subject-matters and preferred embodiments, which respectively taken alone or in combination, contribute to solving the object of the present invention:

1. Process for the preparation of a liquisolid powder comprising 2-{4-[N-(5,6-diphenylpyrazin-2-yl)-N-(isopropylamino]butoxy}-N-(methanesulfonyl)acetamide (selexipag), the process comprising the following steps:

a) providing a solution comprising selexipag and a solvent, wherein said solvent is a non-volatile solvent, a mixture of non-volatile solvents, or a mixture of non-volatile and volatile solvents, to form a solution comprising selexipag;
b) mixing the solution obtained in step a) with a solid porous carrier, thereby obtaining a liquisolid powder;

wherein said solvent has a hydrophilic-lipophilic balance (HLB)-value in a range from 10 to 18, and the solubility of selexipag in said solvent is at least 10 mg/ml at room temperature.

2. A process according to item 1, wherein the amount of carrier, in relation to the amount of solvent, is chosen such that the provided carrier is able to absorb the complete amount of solution.

3. A process according to item 1 or 2, wherein the mixing step b) comprises providing the carrier, and adding the selexipag solution to the carrier, until the solution is completely absorbed.
The addition of selexipag solution can be carried out by any suitable method that is known to a skilled person. Preferably, the selexipag solution is added by spraying the solution onto said carrier, or by dropping said solution onto the carrier. Usually, the mixing step (which is the step of combining the carrier and the selexipag solution) lasts about 2 hours at room temperature.

4. A process according to any one of items 1 to 3, wherein the weight ratio of solution :

solid porous carrier is from 1:2 to 2:1, preferably about 1:1.

5. A process according to any one of the previous items, wherein the solution obtained in step a) consists of selexipag and the solvent.

6. A process according to any one of the previous items, wherein essentially all of the selexipag used in step a) is present in dissolved state.

7. A process according to any one of the previous items, wherein the solvent essentially consists of a non-volatile solvent, or of a mixture of non-volatile solvents.

8. A process according to any one of the previous items, wherein the solvent is a non-volatile solvent, or a mixture of non-volatile solvents; preferably, the solvent is a non-volatile solvent.

9. A process according to any one of the previous items, wherein the HLB-value of the solvent or mixture of solvents is in the range from 12 to 17.

10. A process according to any one of the previous items, wherein the solubility of selexipag in said solvent is at least 12 mg/ml, preferably at least 20 mg/ml, more preferably at least 45 mg/ml.

11. A process according to any of the one of the previous items, wherein the solvent is pharmaceutically acceptable, preferably comprises one selected from surfactants, solubilizers, emulsifier, oils and liquid lipids.

12. A process according to any one of the previous items, wherein the solvent is selected from the group consisting of

polysorbates, such as polysorbate 20 (polyoxyethylene (20) sorbitan monolaureate), polysorbate 40 (polyoxyethylene (20) sorbitan monopalmitate), polysorbate 60 (polyoxyethylene (20) sorbitan monostearate), and polysorbate 80 (polyoxyethylene (20) sorbitan monooleate), preferably polysorbate 20 and polysorbate 80;
polyoxyl n castor oil, with n = 30 to 40, such as n = 35 or 40, preferably n = 35;
polyoxyl n hydrogenated castor oil, with n = 30 to 60, such as 30, 35, 40, or 60, preferably 35; and Caprylocaproyl polyoxyl-8 glycerides / Caprylocaproyl macrogol-8 glycerides.

13. A process according to any one of the previous items, wherein the solvent is selected from the group consisting of polysorbate 20 (polyoxyethylene (20) sorbitan monolaureate); polysorbate 40 (polyoxyethylene (20) sorbitan monopalmitate); polysorbate 60 (polyoxyethylene (20) sorbitan monostearate);
polyoxyl n castor oil, with n = 30 to 40, such as n = 35 or 40, preferably n = 35;
polyoxyl n hydrogenated castor oil, with n = 30 to 60, such as 30, 35, 40, or 60, preferably 35; and Caprylocaproyl polyoxyl-8 glycerides / Caprylocaproyl macrogol-8 glycerides.

14. A process according to any one of the previous items, wherein the solvent is selected from the group consisting of polysorbat 20 (polyoxyethylene (20) sorbitan monolaureate); polyoxyl 35 castor oil; and Caprylocaproyl polyoxyl-8 glycerides / Caprylocaproyl macrogol-8 glycerides.

15. A process according to any one of the previous items, wherein the solid porous carrier has a BET specific surface area of at least 150 $m^2/g$, preferably of at least 200 $m^2/g$, or of at least 300 $m^2/g$, and/or an oil absorbing capacity of at least 1.5 ml/g carrier, preferably of at least 1.8 ml/g carrier, more preferably of at least 2.0 ml/g carrier or 2.2 ml/g carrier, and even more preferably of at least 2.5 ml/g carrier, 3.0 ml/g carrier, or 3.2 ml/g carrier.

16. A process according to any one of the previous items, wherein the solid porous carrier has a pore volume in the range of 1.0 $cm^3/g$ to 3.5 $cm^3/g$, preferably from 1.5 $cm^3/g$ to 3.0 $cm^3/g$, more preferably from 1.5 $cm^3/g$ to 2.5 $cm^3/g$, and even more preferably from 1.5 $cm^3/g$ to 2.0 $cm^3$.
By way of example, Syloid XDP has a pore volume of 1.7 $cm^3/g$.

17. A process according to any one of the previous items, wherein the solid porous carrier is an inorganic carrier, preferably the solid porous carrier is selected from the group consisting of magnesium aluminium metasilicate and silicon dioxide, more preferably the porous carrier is selected from the group consisting of Neusilin (e.g. Neusilin US2, CAS-Number 12511-31-8), Syloid (e.g. Syloid XDP), Parteck SLC500 (which is a silicon dioxide from Merck, CAS-Number 7631-86-9), and Silica.

18. A process according to any one of the previous items, wherein the carrier is amorphous, i.e. shows an amorphous halo, when analysed by XRPD.

19. A process according to any one of the previous items, wherein no drying or evaporation step is carried out for obtaining the liquisolid powder.

20. A process for the preparation of a solid final dosage form comprising the process according to any one of the previous items, wherein the process comprises a step c) of formulating the liquisolid powder obtained in step b) into said final dosage form.

21. A process according to the previous item, wherein step c) comprises a step of adding, preferably mixing, one or more pharmaceutically acceptable excipients to the liquisolid powder.

22. A process according to item 20, wherein step c) comprises performing a direct compression step of the liquisolid powder or the mixture of item 21, optionally followed by carrying out a coating step.

23. A process according to any one of items 20 or 21, wherein step c) further comprises an encapsulation step.

24. A process according to any one of items 20 to 23, wherein the one or more pharmaceutically acceptable excipient(s) is/are selected from the group consisting of fillers, glidants, disintegrants, and lubricants, preferably the tablet core comprises only a filler component, a disintegrant component and/or a lubricant component.
In a preferred embodiment, two types of fillers (two different fillers) are used.
Preferred fillers are mannitol, and maize starch.

25. A process according to item 24, wherein
the filler is selected from the group consisting of microcrystalline cellulose, silicified microcrystalline cellulose, microcrystalline cellulose (MCC), lactose monohydrate, lactose, mannitol, maize starch, starch 1500, and pregelatinized starch; preferably, the filler is selected from the group consisting of mannitol, microcrystalline cellulose (MCC), maize starch, starch 1500, and lactose;
the glidant is selected from the group consisting of silicon dioxide, especially colloidal silica, hydrophobic colloidal silica, talc, magnesium silicate and aluminum silicate, preferably fumed silica or Syloid FP silica, more preferably the glidant is fumed silica (Aerosil ®);
the disintegrant is selected from the group consisting of carmellose calcium, carboxymethylstarch sodium, croscarmellose sodium salt (cellulose carboxymethylether sodium salt, crosslinked), croscarmellose sodium (e.g. Ac-Di-Sol®), starch, such as sodium starch glycolate (e.g. Primojel ®) or corn starch, crosslinked polyvinylpyrrolidone (crospovidone), polyvinylpyrrolidone (PVP), and low-substituted hydroxypropylcellulose, preferably selected from the group consisting of low-substituted hydroxypropylcellulose, PVP, sodium starch glycolate, and croscarmellose sodium; and
the lubricant is selected from the group consisting of stearic acid, talc, sodium stearyl fumarate and magnesium stearate, preferably the lubricant is magnesium stearate or sodium stearyl fumarate.

26. A process according to any one of items 20 to 25, wherein, if the final dosage form is a tablet, in step c) the amount of one or more pharmaceutically acceptable excipients added is such that the weight ratio of the liquisolid powder to the total amount of the one or more pharmaceutically acceptable excipient is in a range from 1:2 to 1:10, preferably in a range from 1:2.5 to 1:6, more preferably in a range from 1:2.8 to 1:3.2.

27. A process according to any one of items 20 to 26, wherein the final solid oral dosage form is a sachet, tablet, or capsule.

28. A process according to any one of items 20 to 27, wherein the weight ratio of solution : solid porous carrier is in the range from 1:2 to 2:1, preferably about 1:1.

29. Liquisolid powder comprising selexipag in dissolved state, a solid porous carrier, and a solvent, wherein
the solid porous carrier has a BET specific surface area of at least 150 $m^2/g$, and/or an oil absorbing capacity of at least 1.5 ml/g carrier; and
the solvent is a non-volatile solvent, a mixture of non-volatile solvents, or a mixture of non-volatile and volatile solvents, and wherein said solvent has a hydrophilic-lipophilic balance (HLB)-value in a range from 10 to 18, and the solubility of selexipag in said solvent is at least 10 mg/ml at room temperature.

30. A liquisolid powder according to item 29, wherein the solid porous carrier has a BET specific surface of at least 200 $m^2/g$, or of at least 300 $m^2/g$, and/or an oil absorbing capacity of at least 1.8 ml/g carrier, preferably of at least 2.0 ml/g carrier or 2.2 ml/g carrier, and even more preferably of at least 2.5 ml/g carrier, 3.0 ml/g carrier, or 3.2 ml/g carrier.

31. A liquisolid powder according to item 29 or 30, wherein said porous solid carrier is as defined in item 17 or 18, and/or wherein said solvent is as defined in any one of items 7 to 14.

32. A liquisolid powder according to any one of items 30 to 32, which consists of selexipag in dissolved state, the solid porous carrier, and a solvent.

33. A liquisolid powder according to any one of items 29 to 32, wherein the weight ratio of solution : solid porous carrier is as defined in item 4.

34. A liquisolid powder according to any one of items 29 to 33, wherein all selexipag is in dissolved state.

35. Final dosage form comprising the liquisolid powder of any one of items 29 to 34, and optionally one or more pharmaceutically acceptable excipient.

36. A final dosage from according to item 35, wherein the one or more pharmaceutically acceptable excipient is as defined in item 24 or 25.

37. A final dosage form according to item 35 or 36, wherein the final dosage form is a solid oral dosage form, such

as a tablet, sachet, or capsule.

38. A final dosage form according to any one of items 35 to 37, wherein, if the final dosage form is a tablet, the weight ratio of solution : solid porous carrier is from 1:2 to 2:1, preferably about 1:1; and/or
wherein the weight ratio of liquisolid powder to the total amount of the one or more pharmaceutically acceptable excipient is in the range from 1 : 4 to 1 : 1, preferably in the range from 1 : 3.5 to 1 : 1, more preferably in the range from 1 : 3 to 1 : 1.

39. Use of the liquisolid powder of any one of items 29 to 34 for the preparation of a dosage from.

40. A use according to item 39, wherein the dosage from is a final solid oral dosage from, preferably a tablet, sachet, or capsule.

41. Liquisolid powder of any one of items 29 to 34, or final dosage form of any one of items 36 to 39, for use in a method of prevention or treatment of pulmonal arterial hypertension.

Definitions

Where the term "comprising" is used in the present description and claims, it does not exclude other elements or steps. For the purposes of the present invention, the term "consisting of" is considered to be a specific embodiment of the term "comprising". If hereinafter a group is defined to comprise at least a certain number of embodiments, this is also to be understood to disclose a group which optionally consists only of these embodiments.

As used herein the term "room temperature" is understood to mean temperatures of about 15 °C to about 25 °C, e.g., 15 °C to 25 °C or exactly 20 °C [see e.g. EU Pharmacopoeia 7.5, 1.2 (2012)].

Where an indefinite or definite article is used when referring to a singular noun e.g. "a" or "an", "the", this includes a plural of that noun unless something else is specifically stated.

In general, the term "liquid medication" refers to liquid lipophilic drugs and drug suspensions or drug solutions of solid poorly water-soluble or water-insoluble drugs in suitable solvent systems. In the present invention, the solid poorly water-soluble drug is selexipag. Further, selexipag is completely dissolved in a suitable solvent system, therefore forming a drug solution. Thus, within the meaning of the present invention, the term "liquid medication" denotes a drug solution of poorly water-soluble selexipag in a suitable solvent system.

In general, the term "liquisolid powder" refers to powdered forms of liquid medications formulated by converting liquid lipophilic drugs, or drug suspensions or solutions of poorly water-soluble or water-insoluble solid drugs in suitable solvent systems into dry-looking, non-adherent, free-flowing powder admixtures by mixing with a suitable carrier. Within the meaning of the present invention, the liquid medication is a selexipag solution, and thus, the liquisolid powder of the present invention is formulated by converting a liquid selexipag solution in suitable solvent systems into dry-looking, non-adherent, free-flowing powder admixtures by mixing with a suitable carrier.

In general, the term "solvent" denotes a substance that dissolves a solute, thereby resulting in a solution. Usually, a solvent is a liquid. In the present invention, the solvent is a non-volatile solvent, a mixture of non-volatile solvents, or a mixture of non-volatile and volatile solvents. The term "non-volatile" as used herein denotes any liquid which, at sea level and standard pressure conditions (i.e. atmospheric pressure equal to 1 atm) possesses a boiling point greater than the boiling point of distilled water, namely a boiling point greater than 100°C.

The term "HLB value" denotes the hydrophilic-lipophilic balance of a substance and thus gives information on the lipophilic or hydrophilic tendency of a substance. The higher the HLB-value, the better the hydrophilicity. The HLB value can be determined by calculating the values for the different regions of the molecule, as described by Griffin in 1949 (Griffin, William C. (1949), "Classification of Surface-Active Agents by 'HLB", Journal of the Society of Cosmetic Chemists, 1(5): 311-26) and 1954 (Griffin, William C. (1954), "Calculation of HLB Values of Non-Ionic Surfactants", Journal of the Society of Cosmetic Chemists, 5 (4): 249-56), and as described by Davies in 1957 *(*Davies JT (1957), "A quantitative kinetic theory of emulsion type, I. Physical chemistry of the emulsifying agent", Gas/Liquid and Liquid/Liquid Interface, Proceedings of the International Congress of Surface Activity, pp. 426-38). The HLB-value of a mixture of solvents can be determined by multiplying the HLB-value of the single solvents with their weight percentages in the mixture and summing up the obtained values.

As a preferred reference, the HLB-value can be determined by using the Griffin's method for non-ionic surfactants as described in the paper of 1954 (Griffin, William C. (1954), "Calculation of HLB Values of Non-Ionic Surfactants", Journal of the Society of Cosmetic Chemists, 5 (4): 249-56):

$$HLB = 20 \cdot M_h / M,$$

where $M_h$ is the molecular mass of the hydrophilic portion of the molecule, and M is the molecular mass of the whole molecule, giving a result on a scale of 0 to 20. An HLB value of 0 corresponds to a completely lipophilic/hydrophobic molecule, and a value of 20 corresponds to a completely hydrophilic/lipophobic molecule.

Solubility of selexipag in a candidate solvent can be determined at room temperature by providing a certain amount of solvent (e.g., 1000 mg) and adding the solid selexipag (e.g., 100 mg) while stirring the candidate solvent for 2 days. If selexipag does not completely dissolve, further candidate solvent is added until selexipag is completely dissolved.

The term "oral solid dosage form" as used herein denotes solid preparations (e.g. tablets) for oral administration each containing a single dose of the API selexipag.

The terms "about", "substantially", or "essentially" in the context of the present invention denote (unless indicated differently) an interval of accuracy that the person skilled in the art will understand to still ensure the technical effect of the feature in question. The term typically indicates deviation from the indicated numerical value of $\pm 10\%$, and preferably $\pm 5\%$.

The term "absorption" as used herein denotes a process in which a liquid (the solution of the present invention) enters a bulk material (the carrier of the present invention). The absorbate molecules (which is the solution) diffuse inside the absorbent (which is the carrier), and absorption of the liquid occurs.

The term "oil absorbing capacity" denotes the amount of solution of the present invention the carrier may take up (absorb), relative to its own weight. The oil absorption capacity can, e.g., be determined as follows: The oil is mixed thoroughly with the sample using a metal spatula. The addition of oil is continued until a thick paste-like mass is formed. The addition of oil is stopped when the paste-like mass has been obtained without any excess oil. The oil volume consumed is recorded and the adsorption capacity is determined.

The term "%" typically means "weight %" unless indicated otherwise herein.

Detailed description of the invention

The present invention is now described in more detail by preferred embodiments and examples, which are however presented for illustrative purpose only and shall not be understood as limiting the scope of the present invention in any way.

The API selexipag exhibits, amongst others, the difficult properties of being poorly water soluble (BCS class IV) and of exhibiting poor bioavailability. These properties make it a demanding task to provide formulations comprising selexipag that exhibit satisfactory properties in the pharmaceutical filed, for instance satisfying properties with regard to dissolution, bioavailability, stability, and/or processability.

Within the context of the present invention it has now unexpectedly been found that a very specific approach for formulating selexipag can be applied to solve the above problems. Unexpectedly, the present inventors were able to develop solutions of selexipag in solvents comprising or essentially consisting of non-volatile solvents that are effective and beneficial for preparing a liquisolid powder. This liquisolid powder is comparably easy to manufacture, as for instance it is not necessary to stabilize a specific solid form or polymorph of selexipag: selexipag is present in its dissolved form.

A characteristic feature of the present invention resides in the use of a solvent having a hydrophilic-lipophilic balance (HLB)-value in a range from 10 to 18. Provided such particular selection of solvent, relevant conditions including adequate dissolution state/degree of selexipag, and properties of the selexipag solution for adequately entering the pores of a carrier are combined. The solvent that can be used can be a mixture of non-volatile and volatile solvents, or a non-volatile solvent. Although the solvent can be a mixture of volatile and non-volatile solvent, this mixture of solvents must not be too volatile to avoid evaporation and precipitation of selexipag. Moreover, the solvent provides for a sufficiently high concentration of selexipag in order to provide a desirable ratio of drug/carrier.

Accordingly, the invention is defined by a process for the preparation of a liquisolid powder, the process comprising the following steps:

a) providing a solution comprising 2-{4-[N-(5,6-diphenylpyrazin-2-yl)-N-(isopropylamino]butoxy}-N-(methanesulfonyl)acetamide (selexipag) and a solvent, wherein said solvent is a non-volatile solvent, a mixture of non-volatile solvents, or a mixture of non-volatile and volatile solvents, to form a solution comprising selexipag;
b) mixing the solution obtained in step a) with a solid porous carrier, thereby obtaining a liquisolid powder;

wherein said solvent has a hydrophilic-lipophilic balance (HLB)-value in a range from 10 to 18, and the solubility of selexipag in said solvent is at least 10 mg/ml at room temperature.

In step a), a solution of selexipag is provided. This can, e.g., be done by dissolving selexipag in a suitable solvent at room temperature. A suitable concentration of selexipag in said solvent is e.g., at least 0.01 g/ml. A possible upper limit of the concentration of selexipag in said solvent is e.g., 0.08 g/ml. Thus, an example of a suitable concentration range of selexipag in said solvent is 0.01 to 0.08 g/ml. In a preferred embodiment, all of the selexipag is dissolved in the

solvent. In other words, it is preferred that all of the selexipag is present in its dissolved state. This further contributes to an improved dissolution profile when compared to a composition comprising the selexipag only partially dissolved. A method for determining whether all of an originally crystalline selexipag has been dissolved is the X-ray powder diffraction analysis, e.g. by applying the following XRPD parameters:

| **XRPD Parameter<br>Instrument: Panalytical MPD X'Pert Pro** | |
|---|---|
| Tension | 45 kV |
| Current | 40 mA |
| Range | 3 - 45°2Th |
| Step size | 0.0084° |
| Time per Step | 60s |
| Sampel holder | Zero background holder / Backloading |
| Incident beam path | 0.02rad soller slits<br>PDS 20mm / 9mm automatic<br>Mask 15mm / 10mm |
| Diffracted beam path | 0.02rad soller slits<br>PDS 20mm / 9mm automatic<br>Ni filter |
| Detector | X'Celerator |

In one embodiment, it is possible that in the solution of step a), in addition to selexipag, there is one or more additional API present. In this case, this one or more additional API is also present in its dissolved state.

It is however also possible that one or more additional API(s) is present in a pharmaceutical composition comprising the liquisolid powder, with this additional API(s) not being present in said powder.

In a preferred embodiment, the solution of step a) consists of selexipag and the solvent. In other words, there is no further API or excipient present in the liquisolid powder.

Selexipag is only poorly soluble in water. Therefore, it is a considerable challenge to find a suitable solvent that can be used for preparing the solution of step a). The suitable solvent can be a non-volatile solvent, a mixture of non-volatile solvents, or a mixture of non-volatile and volatile solvents. If the solvent is a mixture of non-volatile and volatile solvent, the resulting mixture of solvents is essentially non-volatile, despite the presence of a volatile solvent in the mixture of solvents. The volatility of a certain solvent/mixture of solvents can, for example, be determined according to DIN 53170.

It is also possible that step a) comprises steps a-1) and a-2), with step a-1) comprising the step of dissolving selexipag in an appropriate (volatile) solvent, and with step a-2) comprising mixing of the dissolved selexipag of step a-1) with a non-volatile solvent. In another, preferred, embodiment, step a) comprises the use of a non-volatile solvent only, which provides solubility of selexipag of equal to or more than 10 mg/ml.

The maximum amount of volatile solvents in the solution is the amount which still allows avoiding precipitation of selexipag during absorption of the solution by the pores. The absence of solid crystalline selexipag in the final product can determined, e.g., by X-ray powder diffraction analysis. A solid amorphous form of selexipag can be determined e.g., by XRPD.

In a preferred embodiment, the solvent that is used in step a) essentially consists of a non-volatile solvent, or is a mixture of non-volatile solvents. Additionally preferred, the solvent is a non-volatile solvent.

The use of a solvent according to the present invention can further contribute to ensure that the selexipag that is present in the solution of step a) remains in its dissolved state, even if the solution of step a) is mixed with a solid porous carrier according to the present invention, thereby arriving at a liquisolid powder, and even if this liquisolid powder is then subjected to further processing steps. It is important that the selexipag remains in its dissolved state in order to provide the advantages of the present invention, such as improved solubility, dissolution, bioavailability and/or stability. Upon applying the process according to the present invention, the selexipag, being present in form of the liquisolid powder, remains in a liquid state upon storage under defined conditions (temperature is 20-25°C; closed storage in high density polyethylene (HDPE) bottles) for a certain time period of at least 4 weeks, preferably of at least 5 weeks, and more preferably for at least six weeks. "Remains in a liquid state" in this connection means that no crystalline signals in

XRPD occur.

Examples of solvents that can be used in step a) are the following solvents:

polysorbates, such as polysorbate 20 (polyoxyethylene (20) sorbitan monolaureate; also denoted as Kolliphor PS20), polysorbate 40 (polyoxyethylene (20) sorbitan monopalmitate), polysorbate 60 (polyoxyethylene (20) sorbitan monostearate), and polysorbate 80 (polyoxyethylene (20) sorbitan monooleate; also denoted as Tween 80), preferably polysorbate 20 (HLB value 16.7) and polysorbate 80 (HLB value 15);
polyoxyl n castor oil, with n = 30 to 40, such as n = 35 or 40, preferably n = 35 (polyoxyl 35 castor oil is also known as Kolliphor EL or Cremophor EL, with the CAS-number being 61791-12-6, and has an HLB value of 12-14;
polyoxyl n hydrogenated castor oil, with n = 30 to 60, such as 30, 35, 40, or 60, preferably 35; and Caprylocaproyl polyoxyl-8 glycerides / Caprylocaproyl macrogol-8 glycerides (also referred to as Labrasol ALF; HLB value 12).

The HLB value of Cremophor EL is given as a range of values as it is a mixture, with the main component being glycerol polyethylene glycol ricinoleate. In the context of such ranges, the mean value is used. In the case of Cremophor EL, the mean value of the range, i.e. the HLB of Cremophor EL, is 13. Cremophor EL (or Kolliphor EL) it is a nonionic solubilizer and emulsifier made by reacting castor oil with ethylene oxide in a molar ratio of 1 : 35. The main component of Cremophor® EL is glycerol polyethylene glycol ricinoleate. Together with fatty acid esters of polyethylene glycol, this forms the hydrophobic part of the product. The smaller hydrophylic part consists of free polyethylene glycols and ethoxylated glycerol. The CAS Number is 61791-12-6.

In a further embodiment, the solvent is selected from the group consisting of polysorbate 20 (polyoxyethylene (20) sorbitan monolaureate); polysorbate 40 (polyoxyethylene (20) sorbitan monopalmitate); polysorbate 60 (polyoxyethylene (20) sorbitan monostearate);
polyoxyl n castor oil, with n = 30 to 40, such as n = 35 or 40, preferably n = 35;
polyoxyl n hydrogenated castor oil, with n = 30 to 60, such as 30, 35, 40, or 60, preferably 35; and Caprylocaproyl polyoxyl-8 glycerides / Caprylocaproyl macrogol-8 glycerides.

In a preferred embodiment, the solvent is selected from the group consisting of polysorbat 20 (polyoxyethylene (20) sorbitan monolaureate); polyoxyl 35 castor oil; and Caprylocaproyl polyoxyl-8 glycerides / Caprylocaproyl macrogol-8 glycerides.

It has further been found significant within the meaning of the present invention that a suitable solvent has a certain HLB value. The HLB value denotes the lipophilic or hydrophilic tendency of a substrate. This lipophilic or hydrophilic tendency contributes to the solvent's capability of dissolving selexipag. Thus, choosing a solvent exhibiting a suitable HLB value is important for being able to provide a solution comprising, preferably containing, dissolved selexipag. It has been found out that suitable solvents within the meaning of the present invention have a HLB value in a range from 10 to 18. In a preferred embodiment, the HLB value is in the range of from 12 to 17.

It is important that the HLB-value as defined in the present invention is the "total" HLB-value, i.e. the HLB value that is present at the end of step a). In other words, the solvent, or mixture of solvents, that is used in step a) exhibits the HLB-value as defined herein, also in cases where multiple types of solvents (e.g. mixture of volatile and non-volatile solvent, or mixture of volatile solvents) are used for dissolving selexipag, and also in cases where selexipag is in a first step a-1) dissolved in a volatile solvent, followed by a second step a-2) of mixing with non-volatile solvent.

Additionally, it is important to provide a solution that comprises or contains selexipag in a concentration that is sufficient for finally providing pharmaceutical compositions (or dosage forms) that comprise an amount of selexipag that is effective in treating the diseases listed elsewhere herein, and at the same time fulfils the requirements with regard to patients' compliance. In order to ensure patients' compliance, several aspects have to be taken into account, for instance the size of a dosage form (e.g. a tablet or capsule) must not be too big because in this case the patients' compliance (especially of elderly people or toddlers) is comparably low. Thus, in order to ensure that the concentration of selexipag is sufficient, the solubility of selexipag in said solvent is at least 10 mg/ml at room temperature. In a preferred embodiment, the solubility of selexipag in the solvent is at least 12 mg/ml, more preferably at least 20 mg/ml, and most preferred the solubility of selexipag in the solvent is at least 45 mg/ml.

In step b), the solution obtained in step a) is mixed with a solid porous carrier, thereby obtaining the liquisolid powder. When mixing the solution (liquid) with the solid porous carrier, absorption of the solution occurs: The liquid is absorbed in the interior, i.e. the pores, of the solid porous carrier. The selexipag, being in an essentially dissolved state in the solution of step a), remains in essentially dissolved state upon absorption. This contributes to the improved properties of the liquisolid powder that is obtained after step b).

In a preferred embodiment, the weight ratio of solution : solid porous carrier is at least 1:2, preferably from 1:2 to 2:1, more preferably about 1:1. It is important to choose the ratio such that recrystallization is avoided.

In a further embodiment, in addition to, or independent from the above indicated weight ratio, the solid porous carrier has a BET specific surface area of at least 150 $m^2/g$, preferably of at least 200 $m^2/g$, or of at least 300 $m^2/g$, and/or an oil absorbing capacity of at least 1.5 ml/g carrier, preferably of at least 1.8 ml/g carrier, more preferably of at least 2.0

ml/g carrier or 2.2 ml/g carrier, and even more preferably of at least 2.5 ml/g carrier, 3.0 ml/g carrier, or 3.2 ml/g carrier.

In a further preferred embodiment, the solid porous carrier has a pore volume in the range of 1.0 $cm^3$/g to 3.5 $cm^3$/g, preferably from 1.5 $cm^3$/g to 3.0 $cm^3$/g, more preferably from 1.5 $cm^3$/g to 2.5 $cm^3$/g, and even more preferably from 1.5 $cm^3$/g to 2.0 $cm^3$/g.

In a preferred embodiment, the solid porous carrier is an inorganic carrier, preferably the solid porous carrier is selected from the group consisting of magnesium aluminium metasilicate and silicon dioxide, more preferably the porous carrier is selected from the group consisting of Neusilin (e.g. Neusilin US2, CAS-Number 12511-31-8), Syloid (e.g. Syloid® XDP), Parteck SLC500 (which is a silicon dioxide from Merck, CAS-Number 7631-86-9), and Silica. Syloid® XPD silicas are mesoporous, amorphous, silica gel excipients.

Particularly preferred, the solid porous carrier is selected from the group consisting of Syloid (in particular Syloid® XPD Silica), Silica, and Neusilin US2.

When carrying out the process of the present invention, in a preferred embodiment, no drying or evaporation step is carried out. By avoiding drying and/or evaporation steps, it is additionally facilitated that selexipag remains in its dissolved state. This further contributes to providing a liquisolid powder comprising selexipag with this liquisolid powder exhibiting improved properties e.g. with regard to solubility, dissolution profile, bioavailability, or processability.

In a preferred embodiment, essentially all, or all, of the selexipag is in dissolved state in the pores of the carrier. This means, for example, that there are no XRPD detectable amounts of crystalline selexipag (e.g., less than 10 wt.-% based on the total amount of selexipag). It is, however, not excluded that minor amounts of selexipag may be adsorbed on the walls of the pores of the carrier or may be otherwise present in the pores in amorphous form (i.e. not XRPD detectable).

The liquisolid powder that is prepared by applying the process of the present invention can then be used for the preparation of dosage forms. When preparing dosage forms, an additional step c) can be carried out, in addition to steps a) and b) as described elsewhere herein. By doing so, the liquisolid powder obtained in step b), optionally in admixture with further excipients, is formulated into a dosage form, preferably a final dosage form, and more preferably into a solid final dosage form. This additional step c) can comprise adding, preferably mixing, one or more pharmaceutically acceptable excipients to the liquisolid powder. Furthermore, the liquisolid powder or the admixture can be used as it is for compression into tablets or filling into capsules.

Preferably dependent on the formulation technique applied on the envisaged (final) dosage form, and/or on further factors that are readily apparent to a skilled person such as release profile of the selexipag being present in the liquisolid powder, a person skilled in the art is able to choose the respective suitable pharmaceutically acceptable excipient(s). In a preferred embodiment, the one or more pharmaceutically acceptable excipient that is combined with the liquisolid powder of the present invention is preferably selected from the group consisting of fillers, glidants, disintegrants, and lubricants, preferably the tablet core (which is the tablet without a coating, that may be present) comprises only fillers/filler, and/or a disintegrant, and/or a lubricant. In a preferred embodiment, the liquisolid powder is combined with one or more filler(s), preferably two types of fillers, only. The respective pharmaceutically acceptable excipients can be combined with the liquisolid powder preferably by sieving and mixing processes.

In a particular preferred embodiment, the one or more filler(s) is/are selected from the group consisting of microcrystalline cellulose, silicified microcrystalline cellulose, microcrystalline cellulose (MCC), lactose monohydrate, lactose, mannitol, maize starch, starch 1500, and pregelatinized starch; preferably, the filler is selected from the group consisting of mannitol, microcrystalline cellulose (MCC), maize starch, starch 1500, and lactose;

the one or more glidant(s) is/are selected from the group consisting of silicon dioxide, especially colloidal silica, hydrophobic colloidal silica, talc, magnesium silicate and aluminum silicate, preferably fumed silica or Syloid FP silica, more preferably the glidant is fumed silica (Aerosil ®);

the one or more disintegrant(s) is/are selected from the group consisting of carmellose calcium, carboxymethylstarch sodium, croscarmellose sodium salt (cellulose carboxymethylether sodium salt, crosslinked), croscarmellose sodium (e.g. Ac-Di-Sol®), starch, such as sodium starch glycolate (e.g. Primojel ®) or corn starch, crosslinked polyvinylpyrrolidone (crospovidone), polyvinylpyrrolidone (PVP), and low-substituted hydroxypropylcellulose, preferably selected from the group consisting of low-substituted hydroxypropylcellulose, PVP, sodium starch glycolate, and croscarmellose sodium; and

the one or more lubricant(s) is/are selected from the group consisting of stearic acid, talc, sodium stearyl fumarate and magnesium stearate, preferably the lubricant is magnesium stearate or sodium stearyl fumarate.

In one embodiment, the amount of one or more pharmaceutically acceptable excipient(s) added in step c) is such that the weight ratio of the liquisolid powder to the final dosage form is in a range from 1:2 to 1:4, preferably in a range from 1:2.5 to 1:3.5, more preferably in a range from 1:2.8 to 1:3.2.

In a preferred embodiment, the final solid dosage form is a solid oral dosage form, such as a sachet, tablet, or capsule. More preferably, the final dosage form is a tablet.

Suitable techniques that can be used for formulating tablets are known to a person skilled in the art. For instance, suitable pharmaceutically acceptable excipients can be brought into contact with the liquisolid powder of the present

invention, followed by carrying out a compression step. It is possible to carry out a direct compression, which further contributes to an improved production of dosage forms. Direct compression only requires blending the liquisolid powder of the present invention with the necessary pharmaceutically acceptable excipient(s) (such as lubricant), followed by a compression step. Suitable process parameters for direct compression are known to a skilled person. Preferably, the compression force is in a range of 2 to 4 kN.

The obtained dosage forms such as tablets or capsules can be coated, e.g. with enteric coatings (such as coatings from the Eudragit® series), and/or (further) protective coating(s), such as coatings protecting from light or other disadvantageous influences.

If the final solid dosage form is intended to be a capsule or sachet, the respective containers (vials) that are filled with the liquisolid powder can for instance be a gelatine shell (in case the final dosage form is a capsule), or a sachet if the final dosage form is a sachet.

If the final dosage form is a tablet, the weight ratio of the liquisolid powder to the total amount of the one or more pharmaceutically acceptable excipient is in a range from 1:2 to 1:10, preferably in a range from 1:2.5 to 1:6, more preferably in a range from 1:2.8 to 1:3.2.
This ratio further contributes to providing tablets that still exhibit beneficial and useful properties, such as a satisfying content uniformity, good disintegration properties, good dissolution of selexipag, a high hardness and stability.

The present invention further refers to a liquisolid powder comprising selexipag in dissolved state, a solid porous carrier, and a solvent, wherein the solvent is a non-volatile solvent, a mixture of non-volatile solvents, or a mixture of non-volatile and volatile solvents, and wherein said solvent has a hydrophilic-lipophilic balance (HLB)-value in a range from 10 to 18, and the solubility of selexipag in said solvent is at least 10 mg/ml at room temperature.

In a preferred embodiment, the solid porous carrier has a BET specific surface of at least 200 $m^2/g$, or of at least 300 $m^2/g$, and/or an oil absorbing capacity of at least 1.8 ml/g carrier, preferably of at least 2.0 ml/g carrier or 2.2 ml/g carrier, and even more preferably of at least 2.5 ml/g carrier, 3.0 ml/g carrier, or 3.2 ml/g carrier.

In a further preferred embodiment, the porous solid carrier of the liquisolid powder according to the present invention is as defined elsewhere herein, and/or the solvent that is used for obtaining said liquisolid powder (and thus is present in said liquisolid powder) is as defined elsewhere herein.

In a further preferred embodiment, the liquisolid powder of the present invention consists of selexipag in dissolved state, the solid porous carrier, and a solvent. Whether selexipag is present in dissolved state can be assessed by any suitable method that is known to a skilled person, e.g. by X-ray powder diffraction analysis as described herein, by applying the parameters indicated elsewhere herein. A powder sample is placed onto a sample carrier by using a backloading process. Films are inserted into a zero-background holder by using a Kapton tape.
It is a further preferred embodiment that all selexipag that is comprised by the liquisolid powder is in dissolved state.

In one embodiment, the weight ratio of solution : solid porous carrier is at least 1:2, preferably from 1:2 to 2:1, more preferably about 1:1.

The present invention further refers to a final dosage form comprising the liquisolid powder of the present invention. In a preferred embodiment, the final dosage form additionally comprises one or more pharmaceutically acceptable excipients as defined elsewhere herein. Preferably, the final dosage form is a solid oral dosage form, such as a tablet, sachet, or capsule.

If the final dosage form is a tablet, the weight ratio of solution : solid porous carrier is from 1:2 to 2:1, preferably about 1:1; and/or the weight ratio of liquisolid powder to the total amount of the one or more pharmaceutically acceptable excipient is in the range from 1 : 4 to 1 : 1, preferably in the range from 1 : 3.5 to 1 : 1, more preferably in the range from 1 : 3 to 1 : 1.

The present invention also refers to the use of the liquisolid powder of the present invention for the preparation of a dosage from, wherein said dosage form preferably is a final solid oral dosage from such as a tablet, sachet, or capsule.

Finally, the present invention refers to the liquisolid powder of the present invention or the final dosage form of the present invention, for use in a method of prevention or treatment of pulmonal arterial hypertension.

Description of the figures

Fig. 1 shows an XRPD overlay of the liquisolid powder of the present invention, wherein selexipag is dissolved in the solvent Labrasol ALF, and the solid porous carrier is Neusilin US2. The XRPD data were obtained after 6 weeks of storage under the following conditions:

Temperature: 20-25°C
Closed storage in HDPE bottles
Humidity: 30%-40% relative humidity (RH)
The XRPD measurement confirms that even after storage of 6 weeks, no (re-)crystallization has occurred.

Fig. 2 shows an XRPD overlay of the liquisolid powder of the present invention, wherein selexipag is dissolved in the solvent Labrasol ALF, and the solid porous carrier is Syloid XDP. The XRPD data were obtained after 6 weeks of storage under the following conditions:

Temperature: 20-25°C
Closed storage in HDPE bottles
Humidity: 30%-40% relative humidity (RH)
The XRPD measurement confirms that even after storage of 6 weeks, no (re-)crystallization has occurred.

Examples

Example 1:

1.1: Examples of suitable solvents

Table 1 below exemplifies solvents that have been found remarkably effective to provide useful liquisolid powder of selexipag. In this table, the solvent, together with the supplier, the chemical identity, the respective HLB value, and the solubility of selexipag are indicated.

Table 1

| Product (solvent) | Supplier | Chemical identity | HLB value | Solubility of selexipag |
|---|---|---|---|---|
| Tween 80 | | Polysorbat 80 | 15 | > 10mg/g |
| Kolliphor PS20 | | Polysorbat 20 | 16.7 | >10mg/g |
| Kolliphor EL | BASF | Polyoxyl castor oil | 12-14 | >10mg/g |
| Labrasol ALF | Gattefossé | Caprylocaproyl polyoxyl-8 glycerides / Caprylocaproyl macrogol-8 glycerides | 12 | >10mg/g |

1.2: Examples of solvent that are not suitable

Table 2 indicates solvents that are not suitable for being used in the present invention, as these solvents were found not to provide effective liquisolid powder of selexipag.

Table 2

| Product (solvent) | Supplier | Chemical identity | HLB value | Solubility of selexipag |
|---|---|---|---|---|
| Isopropylmyristate | | | 11.5 | <10mg/g |
| Labrafil M 1944CS | Gattefossé | Oleoyl polyoxyl-6 glycerides / Oleoyl macrogol-6 glycerides | 9 | <10mg/g |
| Capryol 90 | Gattefossé | Propylene glycol monocaprylate (type II) | 5 | <10mg/g |

Example 2:

Preparation of a liquisolid formulation comprising selexipag, Labrasol and Syloid XDP

100 mg Selexipag are dissolved in 8.0 g Labrasol ALF (HLB value 12). The API-solution is mixed with 8 g of Syloid XDP for two hours at room temperature. Subsequently, the liquisolid powder is mixed with Maize Starch, Microcrystalline Cellulose and Magnesium stearate and compressed to form tablets of 135 mg (Korsch XP1, compression force: 2 - 4 kN).

| | [mg] |
|---|---|
| | |
| Selexipag | 0,20 |
| Labrasol ALF | 16,00 |
| Syloid XDP | 16,00 |
| Maize Starch | 50,73 |
| MCC | 50,72 |
| MgStearate | 1,35 |
| | |
| SUM | 135,00 |

The final composition was tested using XRPD method as described above. The results obtained are shown in Fig. 2, upper curve.

Example 3:

Preparation of a liquisolid formulation comprising selexipag, Kolliphor EL and Syloid XDP

100 mg Selexipag are dissolved in 8.0 g Kolliphor EL (HLB value 12-14). The API-solution is mixed with 8 g of Syloid XDP for two hours at room temperature.
Subsequently, the liquisolid powder is mixed with Maize Starch, Microcrystalline Cellulose and Magnesium stearate and compressed to form tablets of 135 mg. (Korsch XP1, compression force: 2 - 4 kN).

Example 4:

Preparation of a liquisolid formulation comprising selexipag, Polysorbat 20 and Syloid XDP

100 mg Selexipag are dissolved in 8.0 g Polysorbat 20 (HLB value 16.7). The API-solution is mixed with 8 g of Syloid XDP for two hours at room temperature.
Subsequently, the liquisolid powder is mixed with Maize Starch, Microcrystalline Cellulose and Magnesium stearate and compressed to form tablets of 135 mg. (Korsch XP1, compression force: 2 - 4 kN).

Example 5:

Preparation of a liquisolid formulation comprising selexipag, Labrasol and Neusilin US2

100 mg Selexipag are dissolved in 8.0 g Labrasol ALF (HLB value 12). The API-solution is mixed with 8 g of Neusilin US2 for two hours at room temperature.
Subsequently, the liquisolid powder is mixed with Maize Starch, Microcrystalline Cellulose and Magnesium stearate and compressed to form tablets of 135 mg. (Korsch XP1, compression force: 2 - 4 kN).
The final composition was tested using XRPD method as described above. The results obtained are shown in Fig. 1 upper curve.

Example 6:

Preparation of a liquisolid formulation comprising selexipag, Kolliphor EL and Neusilin US2

100 mg Selexipag are dissolved in 8.0 g Kolliphor EL (HLB value 12-14). The API-solution is mixed with 8 g of Neusilin US2 for two hours at room temperature.
Subsequently, the liquisolid - powder is mixed with Maize Starch, Microcrystalline Cellulose and Magnesium stearate and compressed to form tablets of 135 mg. (Korsch XP1, compression force: 2 - 4 kN).

Example 7:

Preparation of a liquisolid formulation comprising selexipag, Polysorbat 80 and Syloid XDP

100 mg Selexipag are dissolved in 8.0 g Polysorbat 80 (HLB value 15). The API-solution is mixed with 8 g of Syloid XDP for two hours at room temperature.
Subsequently, the liquisolid - powder is mixed with Maize Starch, Microcrystalline Cellulose and Magnesium stearate and compressed to form tablets of 135 mg. (Korsch XP1, compression force: 2 - 4 kN).

Example 8:

The samples of Example 2 and Example 5 were stored at 20-25°C in closed HDPE bottles for 6 weeks. Then, XRPD was measured. The results are shown in Figure 2 (lower curve) and Figure 1 (lower curve).

## Claims

1. Process for the preparation of a liquisolid powder comprising 2-{4-[N-(5,6-diphenylpyrazin-2-yl)-N-(isopropylamino]butoxy}-N-(methanesulfonyl)acetamide (selexipag), the process comprising the following steps:

   a) providing a solution comprising selexipag and a solvent, wherein said solvent is a non-volatile solvent, a mixture of non-volatile solvents, or a mixture of non-volatile and volatile solvents, to form a solution comprising selexipag;
   b) mixing the solution obtained in step a) with a solid porous carrier, thereby obtaining a liquisolid powder;

   wherein said solvent has a hydrophilic-lipophilic balance (HLB)-value in a range from 10 to 18, and the solubility of selexipag in said solvent is at least 10 mg/ml at room temperature.

2. The process according to claim 1, wherein

   (i) the mixing step b) comprises providing the carrier, and adding the selexipag solution to the carrier, until the solution is completely absorbed; and/or
   (ii) the weight ratio of solution : solid porous carrier is from 1:2 to 2:1, preferably about 1:1; and/or
   (iii) the solution obtained in step a) consists of selexipag and the solvent; and/or
   (iv) the HLB-value of the solvent or mixture of solvents is in the range from 12 to 17;
   and/or
   (v) the solubility of selexipag in said solvent is at least 12 mg/ml, preferably at least 20 mg/ml, more preferably at least 45 mg/ml.

3. The process according to claim 1 or 2, wherein

   (i) the solvent is pharmaceutically acceptable, preferably comprises one selected from surfactants, solubilizers, emulsifiers, oils and liquid lipids; and/or
   (ii) the solvent is selected from the group consisting of
   polysorbates, such as polysorbate 20 (polyoxyethylene (20) sorbitan monolaureate), polysorbate 40 (polyoxyethylene (20) sorbitan monopalmitate), polysorbate 60 (polyoxyethylene (20) sorbitan monostearate), and polysorbate 80 (polyoxyethylene (20) sorbitan monooleate), preferably polysorbate 20 and polysorbate 80;
   polyoxyl n castor oil, with n = 30 to 40, such as n = 35 or 40, preferably n = 35;
   polyoxyl n hydrogenated castor oil, with n = 30 to 60, such as 30, 35, 40, or 60, preferably 35; and Caprylocaproyl polyoxyl-8 glycerides / Caprylocaproyl macrogol-8 glycerides.

4. The process according to any one of the previous claims, wherein the solid porous carrier has a BET specific surface area of at least 150 $m^2/g$, and/or an oil absorbing capacity of at least 1.5 ml/g carrier.

5. The process according to any one of the previous claims, wherein

   (i) the solid porous carrier has a pore volume in the range of 1.0 $cm^3/g$ to 3.5 $cm^3/g$; or
   (ii) the solid porous carrier is selected from the group consisting of magnesium aluminium metasilicate and

silicon dioxide.

**6.** The process for the preparation of a solid final dosage form comprising the process according to any one of the previous claims, wherein the process comprises a step c) of formulating the liquisolid powder obtained in step b) into said final dosage form.

**7.** Liquisolid powder comprising selexipag in dissolved state, a solid porous carrier, and a solvent, wherein
the solid porous carrier has a BET specific surface area of at least 150 $m^2/g$, and/or an oil absorbing capacity of at least 1.5 ml/g carrier; and
the solvent is a non-volatile solvent, a mixture of non-volatile solvents, or a mixture of non-volatile and volatile solvents, and wherein said solvent has a hydrophilic-lipophilic balance (HLB)-value in a range from 10 to 18, and the solubility of selexipag in said solvent is at least 10 mg/ml at room temperature.

**8.** The liquisolid powder according to claim 7, wherein

(i) the solid porous carrier has a BET specific surface of at least 200 $m^2/g$, or of at least 300 $m^2/g$, and/or an oil absorbing capacity of at least 1.8 ml/g carrier, preferably of at least 2.0 ml/g carrier or 2.2 ml/g carrier, and even more preferably of at least 2.5 ml/g carrier, 3.0 ml/g carrier, or 3.2 ml/g carrier; and/or
(ii) said porous solid carrier is selected from the group consisting of magnesium aluminium metasilicate and silicon dioxide, more preferably the porous carrier is selected from the group consisting of Neusilin, Syloid, Parteck SLC500, and Silica; and/or
(iii) the liquisolid powder consists of selexipag in dissolved state, the solid porous carrier, and a solvent;
(iv) the weight ratio of solution : solid porous carrier is as defined in claim 3; and/or
(v) the solvent is a non-volatile solvent.

**9.** Final dosage form comprising the liquisolid powder of claim 7 or 8, and optionally one or more pharmaceutically acceptable excipient(s).

**10.** The final dosage from according to claim 9, wherein

(i) the one or more pharmaceutically acceptable excipient(s) is/are selected from the group consisting of fillers, glidants, disintegrants, and lubricants; and/or
(ii) the final dosage form is a solid oral dosage form, such as a tablet, sachet, or capsule;
and/or
(iii) if the final dosage form is a tablet, the weight ratio of solution : solid porous carrier is from 1:2 to 2:1; and/or the weight ratio of liquisolid powder to the total amount of the one or more pharmaceutically acceptable excipient is in the range from 1 : 4 to 1 : 1.

**11.** Use of the liquisolid powder of claim 7 or 8 for the preparation of a dosage from.

**12.** The use according to claim 11, wherein the dosage from is a final solid oral dosage from, preferably a tablet, sachet, or capsule.

**13.** Liquisolid powder of claim 7 or 8, or final dosage form of claim 9 or 10, for use in a method of prevention or treatment of pulmonal arterial hypertension.

Fig. 1

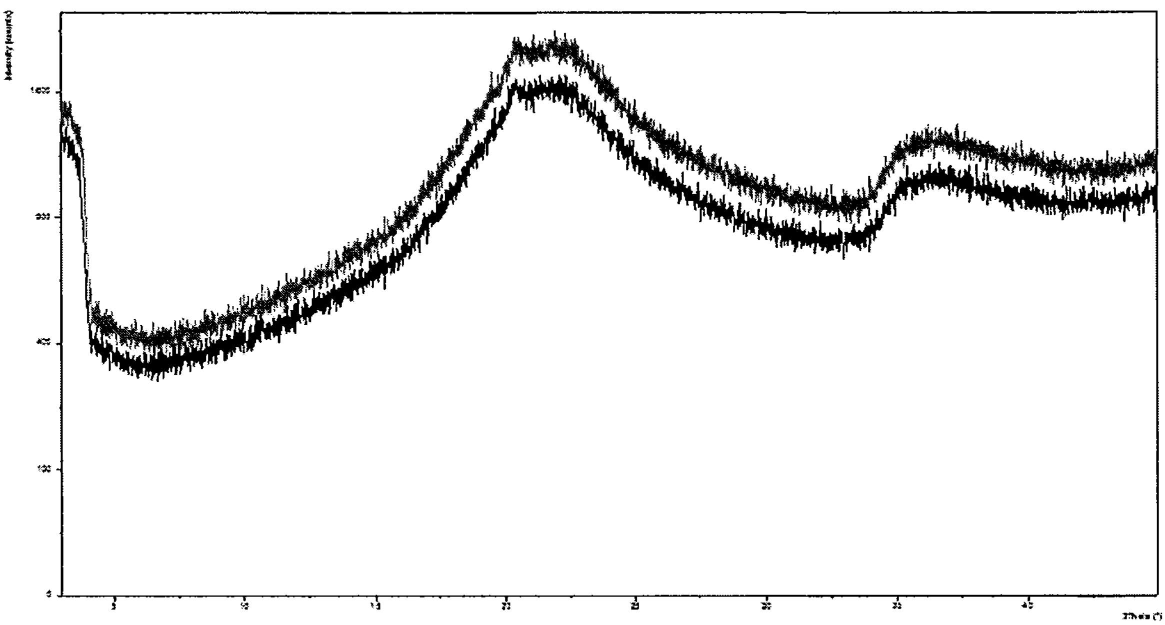

Legend:

upper curve (light grey): samples of Example 5

lower curve (dark grey): sample of Example 5 stored at 20-25°C; in closed HDPE bottles for 6 weeks

Fig. 2

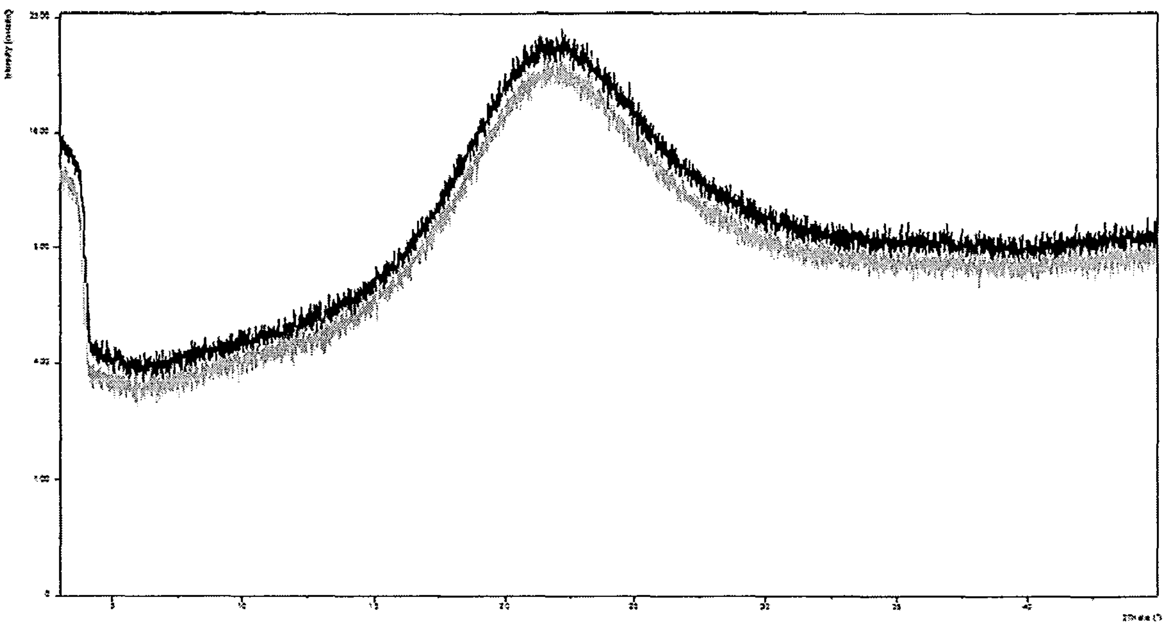

Legend:

upper curve (light grey): samples of Example 2

lower curve (dark grey): sample of Example 2 stored at 20-25°C; in closed HDPE bottles for 6 weeks

Europäisches Patentamt
European Patent Office
Office européen des brevets

# EUROPEAN SEARCH REPORT

Application Number
EP 16 20 4499

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2016/354388 A1 (HARTMAN MICHAEL [US] ET AL) 8 December 2016 (2016-12-08)<br>* paragraph [0001] *<br>* paragraph [0008] - paragraph [0056] *<br>* paragraph [0073] - paragraph [0092] *<br>* paragraph [0110] *<br>* paragraph [0136] - paragraph [0140] *<br>----- | 1-13 | INV.<br>A61K9/14<br>A61K47/02<br>A61K9/16 |
| X | US 2015/196516 A1 (YACOUB MAGDI HABIB [GB] ET AL) 16 July 2015 (2015-07-16)<br>* paragraphs [0002], [0009] *<br>* paragraph [0016] - paragraph [0036] *<br>* paragraph [0089] - paragraph [0091] *<br>* claims 1-3, 8-26 *<br>----- | 1-13 | |
| E | WO 2017/042731 A1 (LUPIN LTD [IN]) 16 March 2017 (2017-03-16)<br>* page 1 *<br>* page 7, line 4 - page 9, line 26 *<br>* examples 2, 3, 6, 7 *<br>* claims 11-20 *<br>----- | 1-13 | |

TECHNICAL FIELDS SEARCHED (IPC)

A61K

The present search report has been drawn up for all claims

1

| Place of search | Date of completion of the search | Examiner |
|---|---|---|
| The Hague | 12 June 2017 | González Ferreiro, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 16 20 4499

This annex lists the patent family members relating to the patent documents cited in the above-mentioned European search report. The members are as contained in the European Patent Office EDP file on
The European Patent Office is in no way liable for these particulars which are merely given for the purpose of information.

12-06-2017

| Patent document cited in search report | Publication date | Patent family member(s) | Publication date |
|---|---|---|---|
| US 2016354388 A1 | 08-12-2016 | AU 2014229265 A1 | 03-09-2015 |
| | | CA 2905615 A1 | 18-09-2014 |
| | | CL 2015002632 A1 | 28-03-2016 |
| | | CN 105188757 A | 23-12-2015 |
| | | EA 201591729 A1 | 31-05-2016 |
| | | EP 2968570 A1 | 20-01-2016 |
| | | HK 1212616 A1 | 17-06-2016 |
| | | JP 2016512494 A | 28-04-2016 |
| | | KR 20150129716 A | 20-11-2015 |
| | | PE 16552015 A1 | 26-11-2015 |
| | | PH 12015501882 A1 | 07-12-2015 |
| | | SG 11201506397Q A | 29-09-2015 |
| | | TN 2015000395 A1 | 03-01-2017 |
| | | US 2014302147 A1 | 09-10-2014 |
| | | US 2016354388 A1 | 08-12-2016 |
| | | WO 2014141135 A1 | 18-09-2014 |
| US 2015196516 A1 | 16-07-2015 | EP 2893922 A1 | 15-07-2015 |
| | | GB 2526897 A | 09-12-2015 |
| | | US 2015196516 A1 | 16-07-2015 |
| WO 2017042731 A1 | 16-03-2017 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 2447254 A


### Non-patent literature cited in the description


- **GRIFFIN, WILLIAM C.** Classification of Surface-Active Agents by 'HLB. *Journal of the Society of Cosmetic Chemists,* 1949, vol. 1 (5), 311-26
- **GRIFFIN, WILLIAM C.** Calculation of HLB Values of Non-Ionic Surfactants. *Journal of the Society of Cosmetic Chemists,* 1954, vol. 5 (4), 249-56
- **DAVIES JT.** A quantitative kinetic theory of emulsion type, I. Physical chemistry of the emulsifying agent. *Gas/Liquid and Liquid/Liquid Interface, Proceedings of the International Congress of Surface Activity,* 1957, 426-38